(19) Europäisches Patentamt
European Patent Office
Office européen des brevets

(11) Publication number: 0 206 772 A2

(12) **EUROPEAN PATENT APPLICATION**

(21) Application number: 86304731.2

(22) Date of filing: 19.06.86

(51) Int. Cl.[4]: C 07 D 241/44, C 07 D 215/22, C 07 D 213/64, C 07 D 253/08, C 07 D 263/58, C 07 D 277/68, A 01 N 43/40, A 01 N 43/42, A 01 N 43/60, A 01 N 43/707, A 01 N 43/74

(30) Priority: 20.06.85 US 746756
07.05.86 US 858681

(43) Date of publication of application: 30.12.86 Bulletin 86/52

(84) Designated Contracting States: AT BE CH DE FR GB IT LI LU NL SE

(71) Applicant: E.I. DU PONT DE NEMOURS AND COMPANY, 1007 Market Street, Wilmington Delaware 19898 (US)

(72) Inventor: Fawzi, Maged Mohamed, 26 Glenbarry Drive, Wilmington Delaware 19808 (US)

(74) Representative: Hildyard, Edward Martin et al, Frank B. Dehn & Co. European Patent Attorneys Imperial House 15-19 Kingsway, London WC2B 6UZ (GB)

(54) **Herbicidal aryloxybenzeneacetic acid derivatives.**

(57) Compounds of the formula

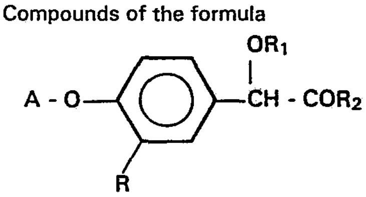

wherein A is specified nitrogen-containing heterocycle;
R is $H_2$ halogen, $CH_3$, $OCH_3$ or $OC_2H_5$;
$R_1$ is $C_1$-$C_3$ alkyl, allyl or propargyl;
$R_2$ is $X_1R_8$ or OH;
$X_1$ is O or S; and
$R_8$ is an organic group; and their agriculturally suitable salts, exhibit herbicidal activity, especially for the selective control of weeds in rice.

The compound may be made by a variety of synthetic routes, e.g. by reacting a haloheterocycle of formula

A - Hal

with an alkali metal salt of the appropriate 4-hydroxy-benzene-acetate ester.

EP 0 206 772 A2

TITLE BA-8585-A

# HERBICIDAL ARYLOXYBENZENEACETIC ACID DERIVATIVES

## Background of the Invention

This invention relates to herbicidally active phenylacetic acid derivatives, agriculturally suitable compositions thereof and a method for their use as herbicides.

The presence of undesirable vegetation causes substantial damage to useful crops, especially agricultural products that satisfy man's basic food and fiber needs, such as cotton, soybeans, rice and the like. The current population explosion and concomitant world food and fiber shortage underlie the need for improvements in the efficiency of producing these crops. Preventing or minimizing the loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides. The need exists, however, for still more effective herbicides that destroy or control weeds without causing significant damage to useful crops.

DE-A-30 04 770, which was published on August 28, 1980, discloses quinoxaline and quinoline compounds of the formula:

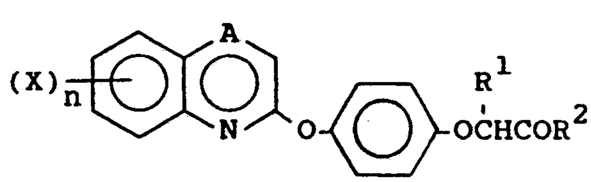

wherein

A is CH or N;

X is halogen;

n is 0, 1 or 2;

$R^1$ is H or lower alkyl;

$R^2$ is OH, alkoxy, OM, $NR^3R^4$, lower alkenyloxy, benzyloxy, alkoxyalkoxy, phenoxy, cyclohexyloxy, haloalkoxy, alkynyloxy or cyanoalkoxy;

M is an organic or inorganic cation;

$R^3$ is H or lower alkyl; and

$R_4$ is H or lower alkyl.

EP-A-23,785, published on February 11, 1981, discloses herbicidal quinoxaline compounds of the general formula:

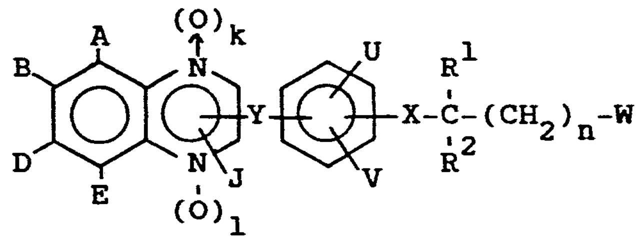

where

A, B, D, E, J, U and V may be halogen, among others;

$R^1$ may be H, $C_1$-$C_6$ alkyl, $C_2$-$C_6$ alkenyl, $C_2$-$C_6$ alkoxyalkyl, $C_1$-$C_6$ haloalkyl, acetyl, propionyl or $C_2$-$C_6$ alkoxycarbonyl;

$R^2$ may be H, $C_1$-$C_6$ alkyl, $C_2$-$C_6$ alkenyl, $C_2$-$C_6$ alkoxyalkyl, $C_1$-$C_6$ haloalkyl, or $R^1$ and $R^2$ taken together may form a methylene, ethylidene, propylidene or isopropylidene group; and

W may be COG and G may be $C_1$-$C_{10}$ alkoxy, $C_2$-$C_{10}$ alkenyloxy, and others.

EP-A-42,750, published on December 30, 1981, discloses herbicidal quinoxaline compounds of general formula:

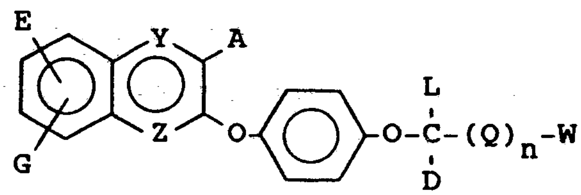

(I)

where

A is H or $C_1$-$C_4$ alkyl;

E is H, Cl, Br, F, $CF_3$, $CH_3$ or $OCH_3$;

G is H or Cl;

D is H, $CH_3$ or $CO_2R_8$;

L is H, $CH_3$ or $C_2H_5$;

Q is -CH=CH- or $-CH_2CH_2-$;

n is 0 or 1;

W is CN or C(O)R or 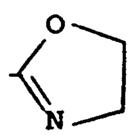 ; and

Y and Z are independently N or N--O.

U.S. Patent 4,236,912, issued to Johnston et al. on December 2, 1980, discloses herbicidal quinolinyloxyphenoxy ethers of the formula

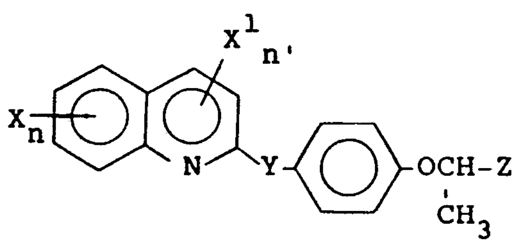

where

X and $X^1$ are independently F, Cl, Br, $CF_3$, $OCH_3$, $NO_2$ or $-N(R^1)_2$, provided that both cannot be $CF_3$, $OCH_3$, $NO_2$ or $-N(R^1)_2$;

n is 0, 1 or 2;

n' is 0 or 1;

Y is O or S; and

Z is $-CO_2H$, $-CO_2M$, $-CO_2R$, $-COSR$, $-CONR^1_2$, $-CSNH_2$, CN, $-CH_2OR^1$ or $-CH_2O_2CR^1$.

Belgian Patent 858,618 discloses herbicidal compounds of the formula

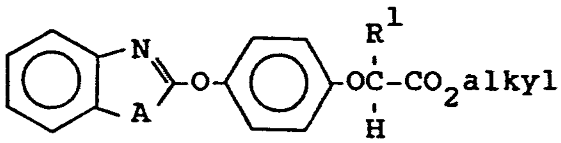

where

A = O, S, N-alkyl, and

$R^1$ = H or $C_1$-$C_4$ alkyl.

U.S. Patent 4,046,553, issued to Takahashi et al. on September 6, 1977, discloses herbicidal compounds of the formula

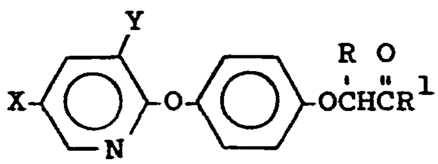

where

X is halogen;

Y is H, halogen or methyl;

R is H or $C_1$-$C_6$ alkyl; and

$R^1$ is hydroxy, $C_1$-$C_9$ alkoxy, $C_2$-$C_4$ alkenyloxy and others.

U.S. Patent 4,309,211, issued to Serban et al. on January 5, 1982, discloses herbicidal compounds of the formula

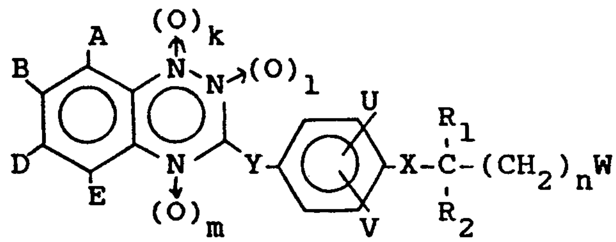

where

$R_1$ is H, alkyl, alkenyl, alkoxyalkyl, haloalkyl acetyl, propionyl or alkoxycarbonyl;

$R_2$ is H, alkyl, alkenyl, alkoxyalkyl or haloalkyl;

A, B, D and E may be halogen; and

W may be C(O)G where G is alkoxy.

## Summary of the Invention

The compounds of the invention are compounds of Formula I

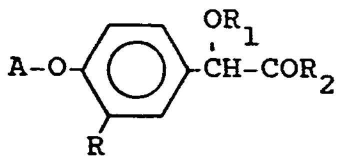

I

where

A is 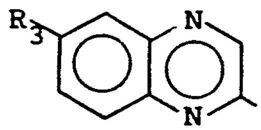, 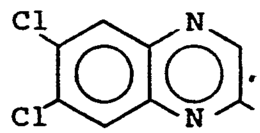, 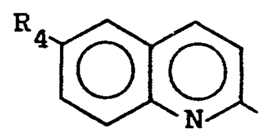,

A-1 A-2 A-3

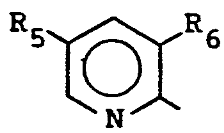, 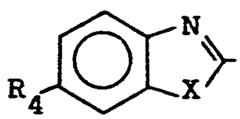 or 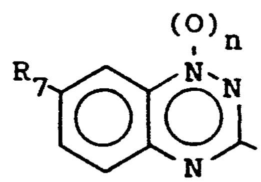;

A-4 A-5 A-6

X is O or S;

n is 0 or 1;

R is H, Cl, F, Br, $CH_3$, $OCH_3$ or $OCH_2CH_3$;

$R_1$ is $C_1$-$C_3$ alkyl, allyl or propargyl;

$R_2$ is $X_1R_8$, OH;

$R_3$ is Cl, F, Br or $CF_3$;

$R_4$ is H, Cl, F or Br;

$R_5$ is Cl or $CF_3$;

$R_6$ is H or Cl;

$R_7$ is H, Cl, F, Br or $CF_3$;

$R_8$ is $C_1$-$C_4$ alkyl, benzyl, phenyl, $C_5$-$C_8$ cycloalkyl, $CH_2CH_2OCH_3$, $CH_2CH_2OCH_2CH_3$, $C_3$-$C_4$ alkenyl, $C_3$-$C_4$ alkynyl, $C_3$-$C_4$ alkenyl substituted with Cl, $CH_2CH_2OCH_2CH_2OCH_3$, $CH_2CH_2OCH_2CH_2OCH_2CH_3$, $CH_2CO_2CH_3$ or $CH(CH_3)CO_2CH_3$;

$X_1$ is O or S;

provided that

a) when $R_5$ is Cl, then $R_6$ is Cl;

b) when A is $A_2$, then R is H;

and their agriculturally suitable salts.

Compounds preferred for their higher herbicidal activity and/or more favorable ease of synthesis are:

1) Compounds of Formula I wherein $R_1$ is $CH_3$ and $R_2$ is $XR_8$.
2) Compounds of Preferred 1 wherein A is A-1, A-3, A-4 or A-5; R is H, Cl, $OCH_3$ or F; and $R_8$ is $C_1$-$C_4$ alkyl.
3) Compounds of Preferred 2 wherein A is A-1.
4) Compounds of Preferred 2 wherein A is A-2.
5) Compounds of Preferred 2 wherein A is A-6.
6) Compounds of Preferred 2 wherein A is A-5.
7) Compounds of Preferred 2 wherein A is A-3.

Compounds specifically preferred for reasons of greatest herbicidal activity and/or most favorable ease of synthesis are:

4-(6-Chloro-2-quinoxalinyloxy)-3-fluoro-α-methoxybenzeneacetic acid, methyl ester, m.p. 101-103°C;

4-(6-Chloro-2-quinoxalinyloxy)-α-methoxybenzeneacetic acid, methyl ester, m.p. 98-100°C;

3-Chloro-4-(6-chloro-2-quinoxalinyloxy)-α-methoxybenzeneacetic acid, methyl ester, m.p. 89-93°C; and

4-(6-Chloro-2-quinoxalinyloxy)-α-3-dimethoxybenzeneacetic acid, methyl ester, m.p. 96-100°C.

## DETAILED DESCRIPTION OF THE INVENTION

Synthesis

I) Compounds of Formula I where $R_1 = C_1$-$C_3$ alkyl and $R_2 = OC_1$-$C_3$ alkyl

The various compounds can be prepared by combining, preferably in equimolar amounts, the haloheterocycle and the alkali metal salt of the alkyl 4-hydroxy-α-alkoxybenzeneacetate as illustrated below using 2,6-dichloroquinoxaline as the haloheterocycle moiety.

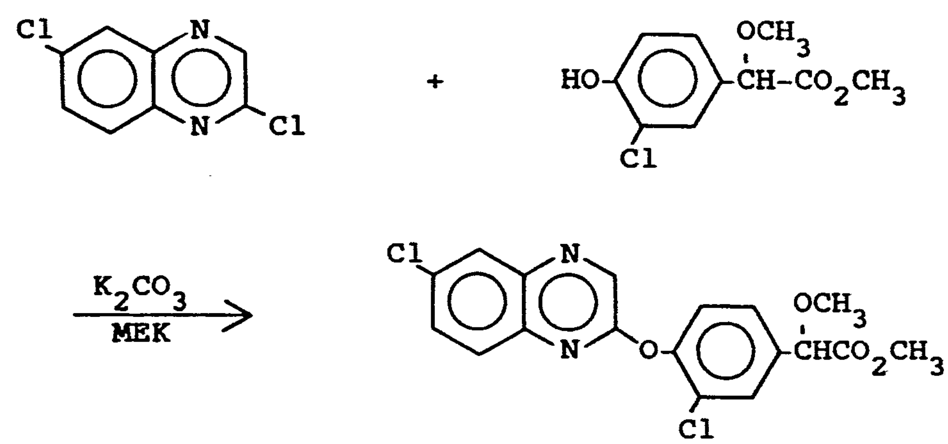


II

Suitable solvent for the reaction include dimethylformamide, dimethylsulfoxide, methyl ethyl ketone and acetonitrile. The reaction is preferably carried out at a temperature between 25 and 100°C.

II) Compounds of Formula I where $R_1 = C_1$-$C_3$ alkyl allyl and propargyl and $R_2 = OC_1$-$C_3$ alkyl

The desired compounds can be prepared from the appropriately substituted alkyl-α-hydroxybenzene-acetates by alkylation with an alkyl halide, allyl bromide or propargyl bromide in the presence of sodium hydride in a suitable solvent such as dimethylformamide, dimethylsulfoxide, tetrahydrofuran or diglyme.

The substituted alkyl α-hydroxybenzeneacetates can be prepared by a modification of the procedure described above where the alkali metal salt of the alkyl α,4-dihydroxybenzeneacetate is used in lieu of the α-alkoxy-4-hydroxy ester in the above example.

The following example clearly illustrates the method employed.

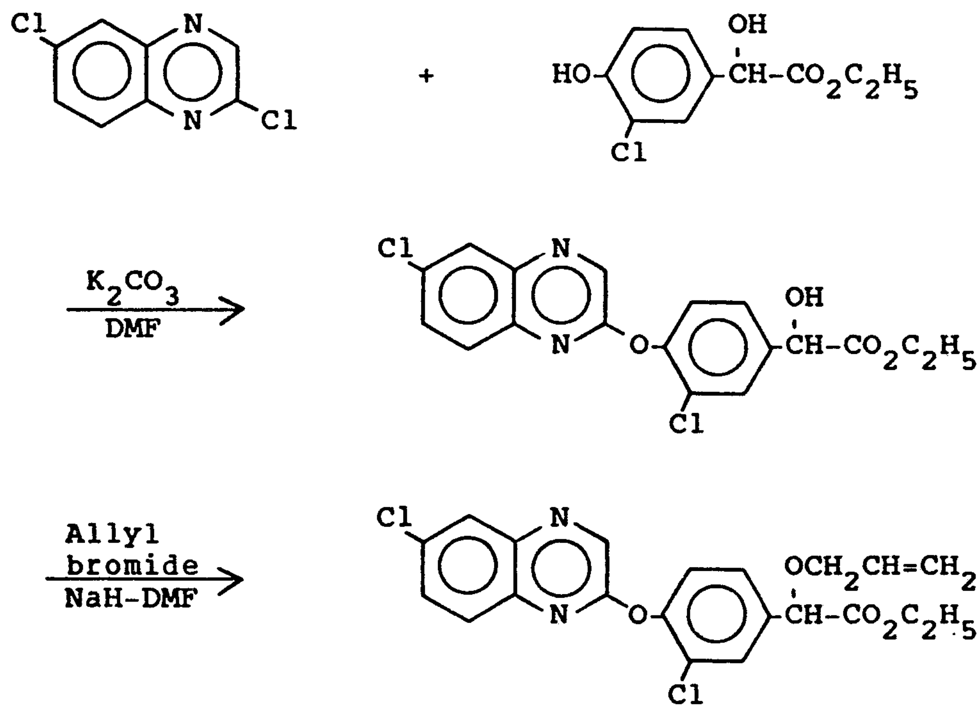

III. Compounds of Formula I where $R_2$ = OH

Hydrolysis of compounds of Formula II where $R_2$ = $OC_1$-$C_3$ alkyl yields the acids. For example,

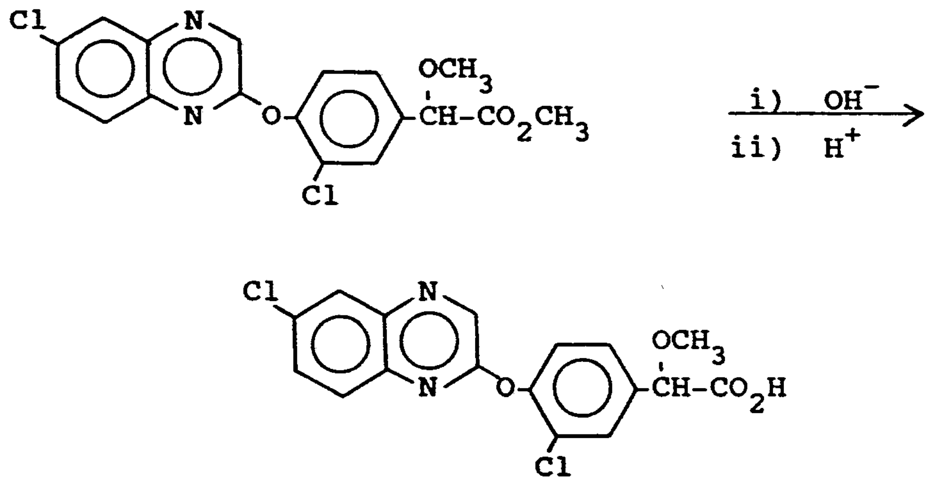


III

IV. Compounds of Formula I where $R_2$ = $X_1R_8$

The acids can be converted to the esters by standard synthetic procedures familiar to those skilled in the art. One of the methods that can be employed is outlined below:

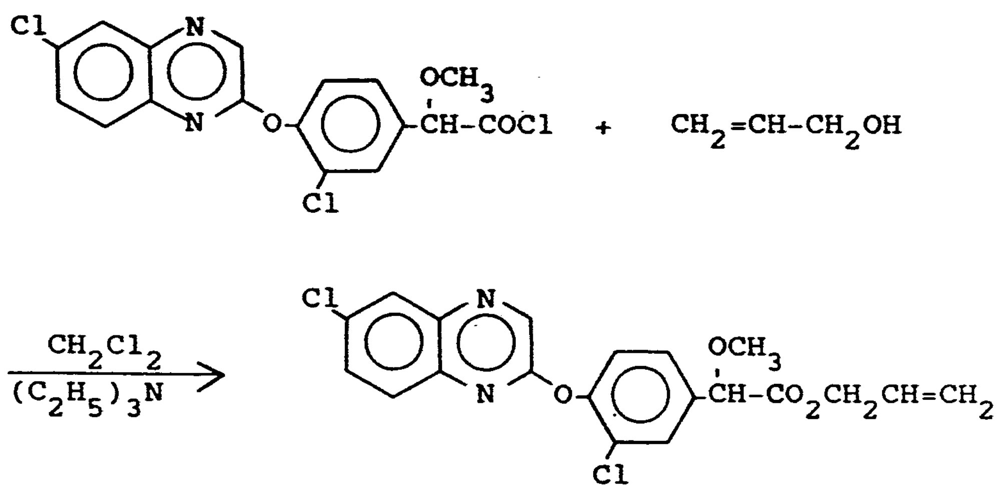

The following Examples illustrate the preparation of some of the compounds of the invention.

Example 1

Methyl 4-(6-chloro-2-quinoxalinyloxy)-3-fluoro-α-methoxybenzeneacetate

In a nitrogen atmosphere, 1.5 g (0.011 mol) potassium carbonate was added to a solution of 1 g (0.005 mol) 2,6-dichloroquinoxaline and 1.1 g (0.005 mol) methyl 3-fluoro-4-hydroxy-α-methoxybenzeneacetate in 50 cc of 2-butanone. The reaction mixture was stirred and refluxed for 3 hours. The hot reaction mixture was filtered and the solid in the filter funnel was washed with 2-butanone. The filtrate and washings were combined and concentrated under vacuum to give the crude product. The product was crystallized from a mixture of *n*-hexane and *n*-chlorobutane (3:1) to give 0.9 g of the title compound, m.p. 101-103°C.

Anal. Calcd for $C_{18}H_{14}ClFN_2O_4$:
C, 57.37; H, 3.72; N, 7.44
Found: C, 57.45; H, 3.88; N, 7.56.

Example 2

Methyl 4-(7-fluoro-1,2,4-benzotriazin-3-yloxy-1-oxide)-α-methoxybenzeneacetate

The named compound was prepared from 3-chloro-7-fluoro-1,2,4-benzotriazine-1-oxide and methyl 4-hydroxy-α-methoxybenzeneacetate by the method described under Example 1. The crude product was crystallized from *n*-chlorobutane, m.p. 115-116°C.

Anal. Calcd for $C_{17}H_{14}FN_3O_5$:
C, 56.82; H, 3.90; N, 11.70
Found: C, 56.95; H, 4.01; N, 11.99.

Example 3

Methyl 4-(6-chloro-2-quinoxalinyloxy)-3-chloro-α-methoxybenzeneacetate

The title compound was prepared from 2,6-dichloroquinoxaline and methyl 3-chloro-4-hydroxy-α-methoxybenzeneacetate by the method described under Example 1. The crude product was purified by flash chromatography (Silica Gel 60, 230-400 mesh ASTM, E. Merck) with n-chlorobutane/ethyl acetate (5:1) as elution solvent, m.p. 89-93°C.

Anal. Calcd for $C_{18}H_{14}Cl_2N_2O_4$:
C, 54.96; H, 3.56; N, 7.12
Found: C, 54.90; H, 3.61; N, 7.00.

Example 4

Methyl 4-(6-chloro-2-quinoxalinyloxy)-α-3-dimethoxybenzeneacetate

The title compound was prepared from 2,6-dichloroquinoxaline and methyl 4-hydroxy-α-3-dimethoxybenzeneacetate by the procedure described under Example 1. The crude product was crystallized from acetonitrile, m.p. 96-100°C.

Anal. Calcd for $C_{19}H_{17}ClN_2O_5$:
C, 58.69; H, 4.38; N, 7.21
Found: C, 58.75; H, 4.45; N, 7.40.

Example 5

a. Ethyl α-allyloxy-4-(6-chloro-2-quinoxalinyloxy)-benzeneacetate

The subject compound can be prepared by the interaction of ethyl 3-chloro-4-(6-chloro-2-quinoxalinyloxy)-α-hydroxybenzeneacetate with allyl bromide in the presence of sodium hydride in dimethylformamide. In a nitrogen atmosphere, add dropwise a solution of 2.3 g (0.0059 mol) ethyl 3-chloro-4-(6-chloro-2-

quinoxalinyloxy)-α-hydroxybenzeneacetate in 60 cc of dimethylformamide at about 0°C to a suspension of 0.23 g of sodium hydride in 10 cc of dimethylformamide. When the evolution of hydrogen ceases, 0.7 g (0.0059 mol) of allyl bromide is added dropwise at 15°C. Stir the mixture for 16 hours and then pour into 100 cc of ice-water. Extract the aqueous solution with methylene chloride (3 X 100 cc). Combine the extracts, wash with water and dry over magnesium sulfate. The magnesium sulfate is removed by filtration and the filtrate is concentrated under vacuum to give the crude product.

b. Ethyl 3-chloro-4-(6-chloro-2-quinoxalinyloxy)-α-hydroxybenzeneacetate

In a nitrogen atmosphere, 3.5 g (0.025 mol) of finely powdered potassium carbonate was added to a solution of 4.6 g (0.023 mol) 2,6-dichloroquinoxaline and 5.4 g (0.023 mol) ethyl 3-chloro-4-hydroxymandelate in 50 cc of dimethylformamide. The reaction mixture was stirred and heated at 65°C for 135 minutes. After standing overnight at room temperature the reaction mixture was poured into 150 cc of ice-water, and the product was extracted into methylene chloride. The methylene chloride solution was washed with water and dried over magnesium sulfate. Removal of the magnesium sulfate by filtration and concentration of the filtrate gave the crude product which was purified by flash chromatography [(Silica Gel 60, 230-400 mesh ASTM, E. Merck) with *n*-chlorobutane/ethyl acetate (20:1) as elution solvent], yield 3.8 g, m.p. 140-145°C.

Anal. Calcd for $C_{18}H_{14}Cl_2N_2O_4$:
C, 54.96; H, 3.56; N, 7.12

Found: C, 54.79; H, 3.65; N, 6.71.

Following the teachings of Examples 1 to 5 and by utilizing the appropriate 2-haloheterocycle and the appropriate alkyl α-alkoxybenzeneacetate or the alkyl halide and the alkyl substituted α-hydroxybenzeneacetate, the ethers in Tables I and II can be prepared.

Table I

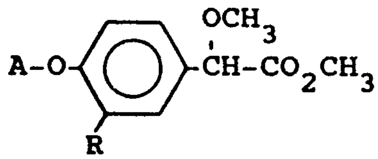

| A | R | m.p.(°C) |
|---|---|---|
| 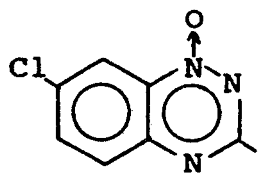 | H | 144-146 |
| 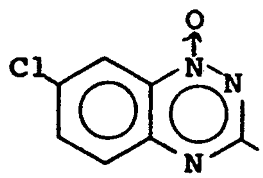 | F | 123-126 |
| 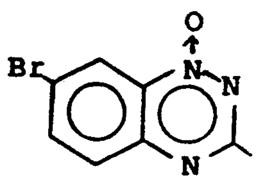 | Cl | 175-178 |
| 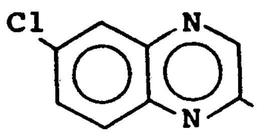 | Br | 107-112 |
| 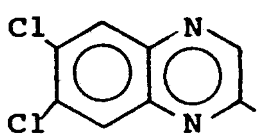 | H | 93-95 |
| 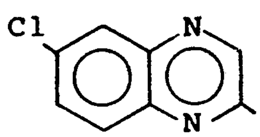 | H | 98-100 |
| 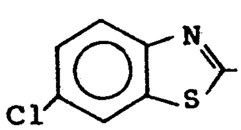 | F | 75-80 |

Table I (continued)

| A | R | m.p.(°C) |
|---|---|---|
| 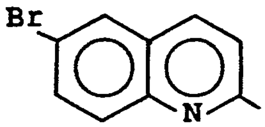 | Cl | Oil<br>NMR ($CDCl_3$)δ3.47 (s, 3H), 3.78 (s, 3H), 4.8 (s, 1H), 7.15-8.1 (m, 8H) |
| 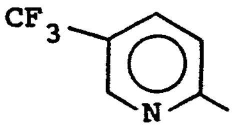 | Cl | Oil<br>NMR ($CDCl_3$)δ3.46 (s, 3H), 3.77 (s, 3H), 4.79 (s, 1H), 7.1 (d, 1H), 7.2 (d, 1H), 7.44 (dd, 1H), 7.6 (d, 1H), 7.9 (dd, 1H), 8.4 (s, 1H) |
| 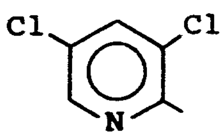 | Cl | Oil<br>NMR ($CDCl_3$)δ3.45 (s, 3H), 3.76 (s, 3H), 4.78 (s, 1H), 7.24 (d, 1H), 7.4 (dd, 1H), 7.6 (d, 1H), 7.79 (d, 1H), 7.91 (d, 1H) |
| 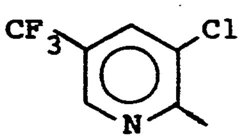 | Cl | Oil<br>NMR ($CDCl_3$)δ3.45 (s, 3H), 3.76 (s, 3H), 4.79 (s, 1H), 7.24 (d, 1H), 7.43 (dd, 1H), 7.61 (d, 1H), 7.99 (d, 1H), 8.22 (d, 1H) |
| 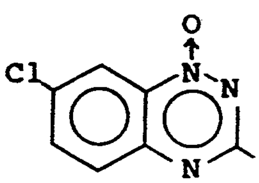 | $OCH_3$ | |
| 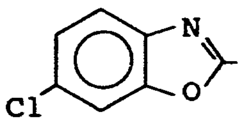 | Cl | |
| 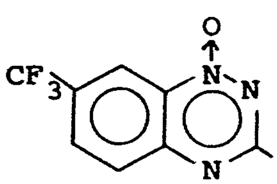 | $OC_2H_5$ | |
| 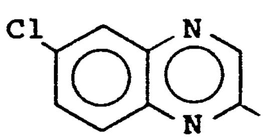 | $CH_3$ | |
| 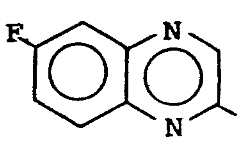 | F | |

## Table II

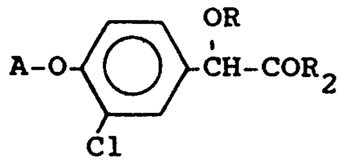

| A | R / $R_2$ | m.p. (°C) |
|---|---|---|
| 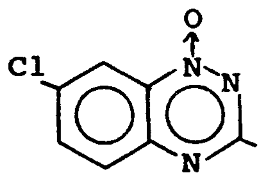 | $(CH_3)_2CH-$ / $(CH_3)_2CHO-$ | 100-103 |
| 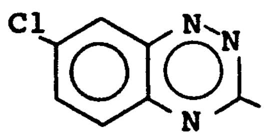 | $C_2H_5-$ / $C_2H_5O-$ | 66-69 |
| 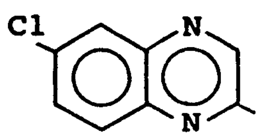 | $(CH_3)_2CH-$ / $(CH_3)_2CHO-$ | 69-75 |
| 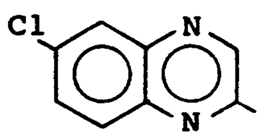 | $HC{\equiv}CCH_2-$ / $CH_3O$ | |

Example 6

3-Chloro-4-(6-chloro-2-quinoxalinyloxy)-α-methoxybenzeneacetic acid

At room temperature, 10 cc of 2N sodium hydroxide were added to a stirred solution of 7.9 g (0.02 mol) methyl 4-(6-chloro-2-quinoxalinyloxy)-3-chloro-α-methoxybenzeneacetate in 50 cc of dimethylformamide and 15 cc of water.

The reaction mixture was stirred at ambient temperature for 30 hours, then acidified to pH 2 by pouring it into ice-hydrochloric acid (ca. 250 cc) to give a gummy semisolid. After decanting the aqueous phase, the semisolid was triturated with *n*-chlorobutane to give a solid which was crystallized from *n*-chlorobutane to give 5.4 g of the title compound, m.p. 128-134°C.

The analytical sample was recrystallized from nitromethane, m.p. 130-135°C.

Anal. Calcd. for $C_{17}H_{12}Cl_2N_2O_3$:
C, 53.83, H, 3.17, N, 7.38
Found C, 53.86, H, 3.24, N, 7.05.

Following the procedure described above and by utilizing the appropriate ester, the acids in the following table can be prepared.

## Table III

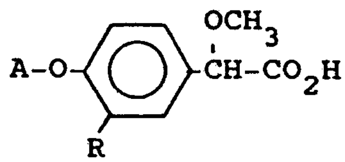

| A | R |
|---|---|
| 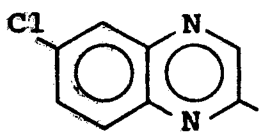 | $OCH_3$ |
| 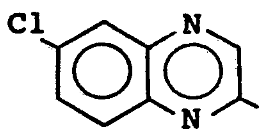 | F |
| 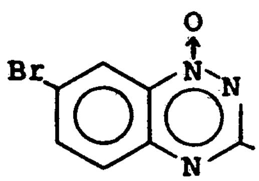 | Cl |
| 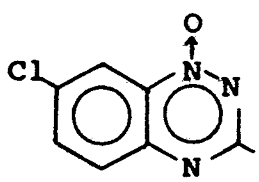 | Cl |
| 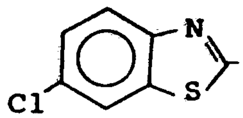 | F |
| 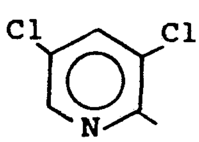 | Cl |
| 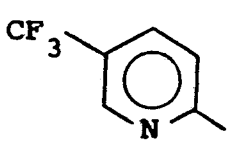 | Cl |

Table III (continued)

| A | R |
|---|---|
| 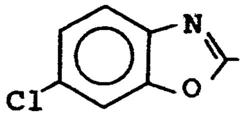 | H |
| 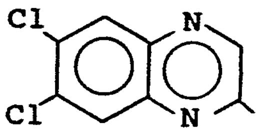 | H |
| 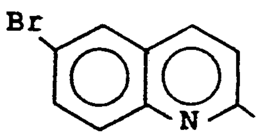 | Cl |

## Example 7

2-(2-Ethoxyethoxy)ethyl-4-(6-chloro-2-quinoxalinyloxy)-3-fluoro-α-methoxybenzeneacetate

The following procedure can be employed to prepare the desired compound.

Add a solution of 3.8 g (0.01 mol) 4-(6-chloro-2-quinoxalinyloxy)-3-fluoro-α-methoxybenzeneacetyl chloride in 30 cc of methylene chloride to a cold (0°C) solution of 1.4 (0.01 mol) 2-(2-ethoxyethoxy)-ethanol and 0.8 g of pyridine in 30 cc of methylene chloride. Stir the mixture at room temperature overnight. Wash the methylene chloride solution with water once and dry the methylene chloride solution over magnesium sulfate. Concentrate the methylene chloride solution under vacuum to give the title compound.

By treatment of the appropriate acid chloride with the appropriate alcohol, thiol, phenol, thiophenol as described above, the compounds listed in Table IV can be made.

Table IV

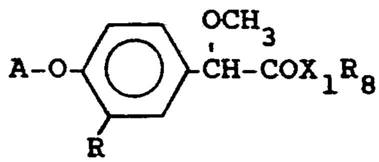

| A | R | $X_1R_8$ |
|---|---|---|
| 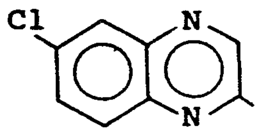 | H | $-O-\overset{CH_3}{CH}-CO_2CH_3$ |
| 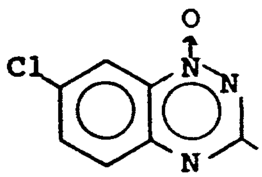 | Cl | -O- 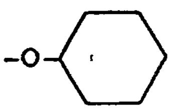 |
| 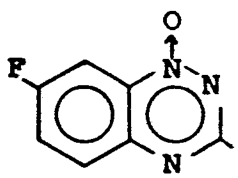 | H | $-OCH_2CH=CHCH_2Cl$ |
| 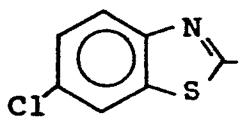 | H | O- 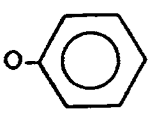 |
| 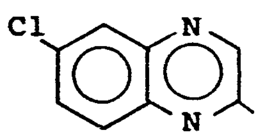 | Cl | $-SCH_2-$ 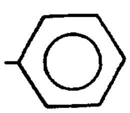 |
| 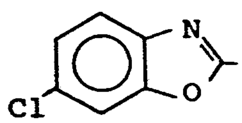 | H | $OCH_2CH_2OC_2H_5$ |
| 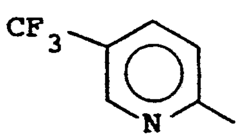 | Cl | $-OC_4H_9$ |

Table IV (continued)

| A | R | $X_1R_{10}$ |
|---|---|---|
| 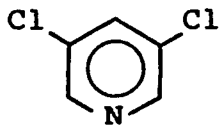 | H | $-OCH_2C{\equiv}CH$ |
| 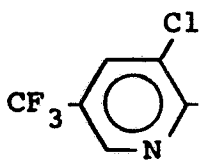 | $OCH_3$ | $OCH_2CH_2OC_2H_5$ |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table V

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a surfactant or a diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

## Example 8

Wettable Powder

| | |
|---|---|
| 4-(6-Chloro-2-quinoxalinyloxy)-3-fluoro-α-methoxy-benzeneacetic acid, methyl ester | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 9

Wettable Powder

| | |
|---|---|
| 4-(6-Chloro-2-quinoxalinyloxy)-α-methoxybenzeneacetic acid, methyl ester | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 10

Granule

| | |
|---|---|
| Wettable Powder of Example 9 | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

Example 11

Extruded Pellet

| | |
|---|---|
| 4-(6-Chloro-2-quinoxalinyloxy)-3-fluoro-α-methoxybenzeneacetic acid, methyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve

(0.42 mm openings) may be packaged for use and the fines recycled.

Example 12

Low Strength Granule

| | |
|---|---|
| 4-(6-Chloro-2-quinoxalinyloxy)-α-methoxybenzene-acetic acid, methyl ester | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 13

Granule

| | |
|---|---|
| 4-(6-Chloro-2-quinoxalinyloxy)-3-fluoro-α-methoxy-benzeneacetic acid, methyl ester | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 14

Low Strength Granule

| | |
|---|---|
| 4-(6-Chloro-2-quinoxalinyloxy)-α-methoxybenzene-acetic acid, methyl ester | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 15

Aqueous Suspension

| | |
|---|---|
| 4-(6-Chloro-2-quinoxalinyloxy)-α-methoxybenzene-acetic acid, methyl ester | 40.0% |
| polyacrylic acid thickener | 0.3% |
| dodecylphenol polyethylene glycol ether | 0.5% |
| disodium phosphate | 1.0% |
| monosodium phosphate | 0.5% |
| polyvinyl alcohol | 1.0% |
| water | 56.7% |

The ingredients are blended and ground together in a sand mill to produce particles essentially all under 5 microns in size.

Example 16

Solution

| | |
|---|---|
| 4-(6-Chloro-2-quinoxalinyloxy)-3-fluoro-α-methoxy-benzeneacetic acid, methyl ester, sodium salt | 5% |
| water | 95% |

The salt is added directly to the water with stirring to produce the solution, which may then be packaged for use.

Example 17

High Strength Concentrate

| | |
|---|---|
| 4-(6-Chloro-2-quinoxalinyloxy)-α-methoxybenzene-acetic acid, methyl ester | 99.0% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 18

Wettable Powder

| | |
|---|---|
| 4-(6-Chloro-2-quinoxalinyloxy)-3-fluoro-α-methoxy-benzeneacetic acid, methyl ester | 90.0% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 19

Wettable Powder

| | |
|---|---|
| 4-(6-Chloro-2-quinoxalinyloxy)-3-fluoro-α-methoxy-benzeneacetic acid, methyl ester | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 20

Oil Suspension

| | |
|---|---|
| 4-(6-Chloro-2-quinoxalinyloxy)-α-methoxybenzene-acetic acid, methyl ester | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 21

Dust

| | |
|---|---|
| 4-(6-Chloro-2-quinoxalinyloxy)-3-fluoro-α-methoxy-benzeneacetic acid, methyl ester | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 22

Oil Suspension

| | |
|---|---|
| 4-(6-Chloro-2-quinoxalinyloxy)-α-methoxybenzene-acetic acid, methyl ester | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 23

Wettable Powder

| | |
|---|---|
| 4-(6-Chloro-2-quinoxalinyloxy)-α-methoxybenzene-acetic acid, methyl ester | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Utility

Test results show that the compounds of the present invention are active herbicides. They are useful for the selective pre- or postemergence weed control in crops, especially in rice, cotton, sugar beets and soybeans.

The rates of application for the compounds of the invention are determined by a number of factors, including the crop species involved, the types of weeds to be controlled, weather and climate, formulations selected, mode of application, amount of foliage present, etc. In general terms, the subject compounds should be applied at levels of around 0.03 to 10 kg/ha, the lower rates being suggested for use on lighter soils and/or those having a low organic matter content or for situations where only short-term persistence is required or for situations where the weed species are easily controlled postemergence.

The compounds of this invention are particularly useful for the control of weeds in rice. They may be used for both paddy and dryland rice. They may be applied postemergence to dryland rice. They may also be applied to paddy rice after transplanting as a spray or granule. The application may be made from 0 to 2 leaf stage of barnyardgrass growth.

Rates of 0.03 to 1 kg/ha will provide weed control. The rate selected for use will depend on the method and timing of application, chemical used, size of weeds, soil type, and other factors. One with ordinary skill in the art can select the rate for any given situation. The compounds are particularly useful for the control of barnyardgrass (Echinochloa crusgalli), a pernicious weed in rice culture, but may also provide complete or partial control of other weeds, particularly gramineous weeds, in rice.

The compounds of the invention may be used in combination with any other commercial herbicide, examples of which are those of the triazine, triazole, uracil, urea, amide, diphenylether, carbamate, bipyridylium and sulfonylurea types.

In particular, the compounds of this invention may be mixed with other rice herbicides to provide a broader spectrum of weed control, extended control or other benefits. They are especially useful for use in conjunction with sulfonylureas selective in rice such as 2-[[(4,6-dimethoxypyrimidin-2-yl)aminocarbonyl]-aminosulfonylmethyl]benzoic acid, methyl ester, 5-[[(4,6-dimethoxypyrimidin-2-yl)aminocarbonyl]aminosulfonyl]-1-methylpyrazole-4-carboxylic acid, ethyl ester, N-[(4,6-dimethoxy-2-pyrimidinyl)aminocarbonyl]-3-(pentafluoro-1-propenyl)-2-thiophenesulfonamide, 3-(1-chloro-2,2-difluoroethenyl-N-[(4,6-dimethoxy-2-pyrimidinyl)aminocarbonyl]-2-thiophenesulfonamide, 3-chloro-5-[[(4,6-dimethoxy-2-pyrimidinyl)aminocarbonyl]aminosulfonyl]-1-methyl-1H-pyrazole-4-carboxylic acid, methyl ester and 3-chloro-5-[[(4-methoxy-6-methylpyrimidin-2-yl)aminocarbonyl]aminosulfonyl]-1-methyl-1H-pyrazole-4-carboxylic acid, methyl ester.

The herbicidal properties of the subject compounds were discovered in a greenhouse test. The test procedures and results follow.

## Compounds

Compound 1

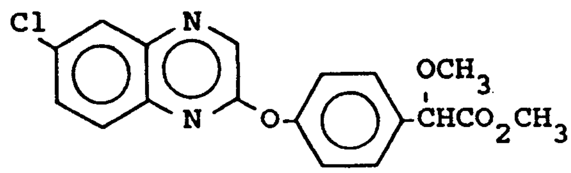

Compound 2

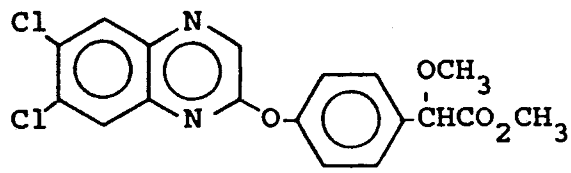

Compound 3

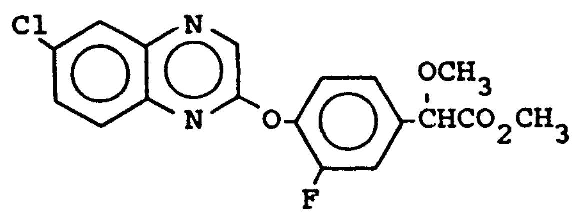

Compound 4

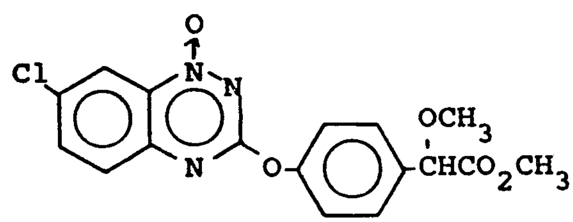

Compound 5

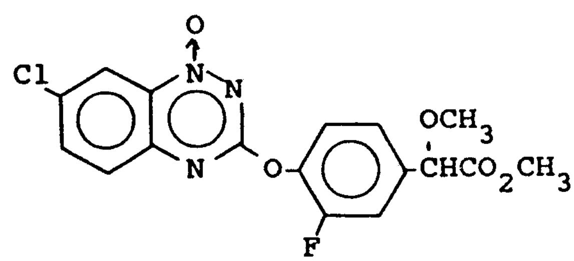

Compound 6

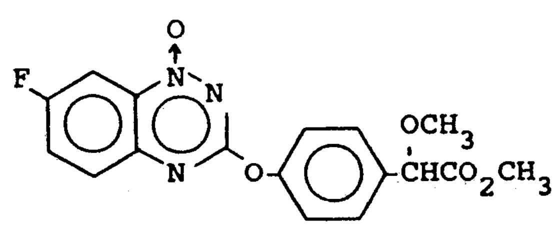

Compounds (continued)

Compound 7

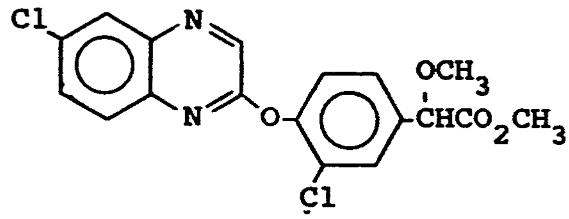

Compound 8

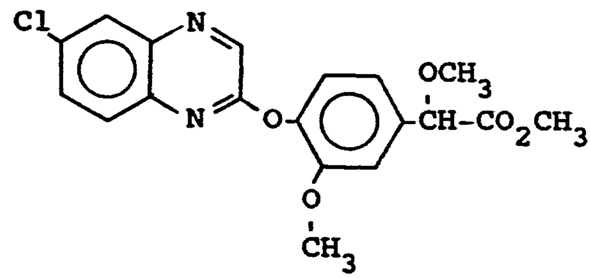

Compound 9

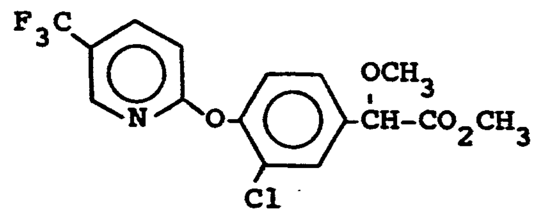

Compound 10

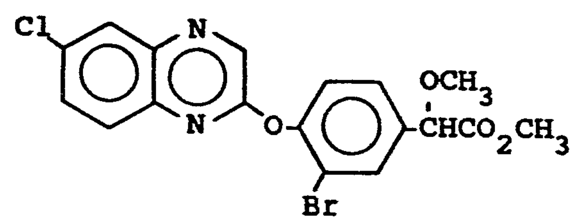

Compound 11

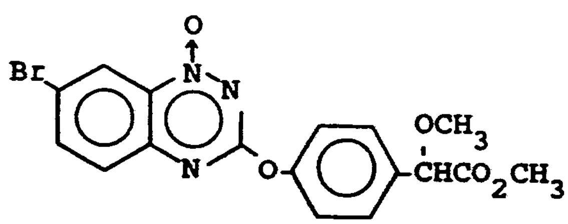

Compound 12

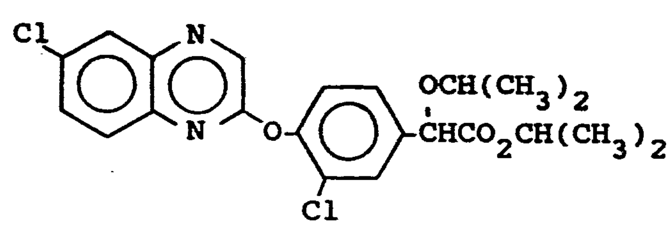

Compounds (continued)

Compound 13

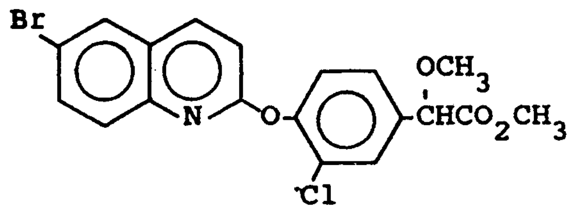

Compound 14

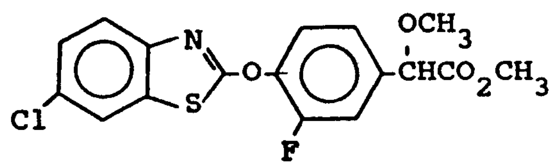

Test A

Seeds of crabgrass (*Digitaria* sp.), barnyardgrass (*Echinochloa crusgalli*), wild oats (*Avena fatua*), sicklepod (*Cassia obtusifolia*), velvetleaf (*Abutilon theophrasti*), morningglory (*Ipomoea* spp.), cocklebur (*Xanthium Pensylvanicum*), sorghum, corn, soybean, sugarbeet, cotton, rice, wheat and purple nutsedge (*Cyperus rotundus*) tubers were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

B = burn;
C = chlorosis/necrosis;
D = defoliation;
E = emergence inhibition;
G = growth retardation;
H = formative effect;
U = unusual pigmentation;
X = axillary stimulation;
S = albinism; and
6Y = abscised buds or flowers.

Table A

| | Cmpd. 1 | Cmpd. 2 | Cmpd. 3 |
|---|---|---|---|
| Rate (kg/ha) | 2 | 2 | 2 |
| POSTEMERGENCE | | | |
| Morningglory | 8B | 5B | 2B |
| Cocklebur | 0 | 0 | 2B |
| Velvetleaf | - | - | 1B |
| Nutsedge | 0 | 0 | 0 |
| Crabgrass | 9C | 2B | 2C,8G |
| Barnyardgrass | 10C | 4B | 10C |
| Cheatgrass | - | - | 0 |
| Wild Oats | 2C,8G | 1B | 8C |
| Wheat | 3C,8G | 1B | 3C,8G |
| Corn | 10C | 2B | 9C |
| Soybean | 1B | 4B | 0 |
| Rice | 9C | 1B | 1C,2H |
| Sorghum | 9C | 1B | 2C,7G |
| Sugar beets | 0 | 1B | 0 |
| Cotton | 1B | 1B | 0 |
| Cassia | 1B | 1B | - |
| PREEMERGENCE | | | |
| Morningglory | 0 | 2G | 0 |
| Cocklebur | - | - | 0 |
| Velvetleaf | - | - | 0 |
| Nutsedge | 7G | 0 | 0 |
| Crabgrass | 9G | 7G | 3C,8G |
| Barnyardgrass | 10E | 5C,9H | 10E |
| Cheatgrass | - | - | 9G |
| Wild Oats | 9G | 2G | 3C,9G |
| Wheat | 9H | 5G | 9C |
| Corn | 2U,9H | 5G | 3C,8H |
| Soybean | 0 | 0 | 0 |
| Rice | 10E | 5G | 4C,8H |
| Sorghum | 10E | 2G | 10E |
| Sugar beets | 5G | 0 | 0 |
| Cotton | 0 | 0 | 0 |
| Cassia | 0 | 0 | - |

Table A (continued)

| | Cmpd. 4 | Cmpd. 5 | Cmpd. 6 |
|---|---|---|---|
| Rate (kg/ha) | 2 | 2 | 2 |
| POSTEMERGENCE | | | |
| Morningglory | 0 | 1B | 1B |
| Cocklebur | 0 | 1B | 3B |
| Velvetleaf | – | 1B | 1B |
| Nutsedge | 0 | 0 | 0 |
| Crabgrass | 5C,9G | 1B | 1B,7G |
| Barnyardgrass | 2C | 3B | 5B |
| Cheatgrass | – | 1B | 1B |
| Wild Oats | 0 | 0 | 0 |
| Wheat | 0 | 0 | 0 |
| Corn | 0 | 2B | 3B |
| Soybean | 1B | 2B | 3B |
| Rice | 2G | 1B,5G | 1B,2G |
| Sorghum | 1B,2G | 1B | 2B |
| Sugar beets | 0 | 0 | 1B |
| Cotton | 1B | 1B | 2B |
| Cassia | 0 | – | – |
| PREEMERGENCE | | | |
| Morningglory | 0 | 0 | 3G |
| Cocklebur | – | 0 | 0 |
| Velvetleaf | – | 0 | 0 |
| Nutsedge | 0 | 0 | 0 |
| Crabgrass | 10E | 10E | 5C,9G |
| Barnyardgrass | 10E | 10E | 10E |
| Cheatgrass | – | 3G | 8G |
| Wild Oats | 9G | 0 | 7G |
| Wheat | 8G | 2G | 5G |
| Corn | 9G | 2C,8G | 2C,9H |
| Soybean | 0 | 0 | 2G |
| Rice | 10E | 1C,5G | 3C,7G |
| Sorghum | 9G | 2C,8G | 4C,9H |
| Sugar beets | 2G | 0 | 9G |
| Cotton | 0 | 0 | 0 |
| Cassia | 0 | – | – |

Table A (continued)

| | Cmpd. 7 | Cmpd. 8 | Cmpd. 9 |
|---|---|---|---|
| Rate kg/ha | 2 | 2 | 2 |
| POSTEMERGENCE | | | |
| Morningglory | 0 | 0 | 0 |
| Cocklebur | 0 | 0 | 0 |
| Velvetleaf | 0 | 4G | 0 |
| Nutsedge | 0 | 0 | 0 |
| Crabgrass | 2G | 9C | 0 |
| Giant Foxtail | 3C,8G | 10C | 0 |
| Barnyardgrass | 8G | 10C | 3S,6H |
| Cheatgrass | 3G | 3S,8G | 0 |
| Wild Oats | 4G | 4C,9G | 0 |
| Wheat | 5G | 4C,9G | 0 |
| Corn | 5H,9G | 3C,9G | 1S,3H |
| Barley | 4C,8G | 3C,9G | 0 |
| Soybean | 3C | 2G | 1H |
| Rice | 2C | 3C,9G | 3S,6G |
| Sorghum | 3G | 3C,8G | 6G |
| Sugar beet | 2G | 0 | 0 |
| Cotton | 0 | 0 | 0 |
| PREEMERGENCE | | | |
| Morningglory | 0 | 0 | 0 |
| Cocklebur | 0 | 0 | 4G |
| Velvetleaf | 0 | 0 | 0 |
| Nutsedge | 0 | 0 | 0 |
| Crabgrass | 5G | 6G | 2C |
| Giant Foxtail | 10E | 9C | 3G |
| Barnyardgrass | 10E | 10E | 9C |
| Cheatgrass | 8G | 0 | 0 |
| Wild Oats | 6G | 0 | 0 |
| Wheat | 7G | 2C,8G | 0 |
| Corn | 8G | 3S,7G | 2G |
| Barley | 6G | 5G | 2C |
| Soybean | 0 | 0 | 0 |
| Rice | 8G | 6S | 2C,5G |
| Sorghum | 8G | 10H | 5G |
| Sugar beet | 8G | 0 | 0 |
| Cotton | 7G | 0 | 9G |

Table A (continued)

| | Cmpd. 10 | Cmpd. 11 | Cmpd. 12 |
|---|---|---|---|
| Rate kg/ha | 2 | 2 | 2 |
| POSTEMERGENCE | | | |
| Morningglory | 1B | 0 | 0 |
| Cocklebur | 0 | 0 | 0 |
| Velvetleaf | 0 | 0 | 7G |
| Nutsedge | 0 | 0 | 0 |
| Crabgrass | 0 | 0 | 0 |
| Giant Foxtail | 0 | 0 | 0 |
| Barnyardgrass | 9B | 0 | 0 |
| Cheatgrass | 0 | 0 | 0 |
| Wild Oats | 0 | 0 | 0 |
| Wheat | 0 | 0 | 0 |
| Corn | 1B | 0 | 0 |
| Barley | 0 | 0 | 0 |
| Soybean | 2B | 0 | 0 |
| Rice | 0 | 0 | 0 |
| Sorghum | 0 | 0 | 0 |
| Sugar beet | 0 | 0 | 0 |
| Cotton | 0 | 0 | 0 |
| PREEMERGENCE | | | |
| Morningglory | 0 | 0 | 0 |
| Cocklebur | – | 0 | 0 |
| Velvetleaf | 0 | 0 | 0 |
| Nutsedge | 0 | 0 | 0 |
| Crabgrass | 0 | 4G | 7G |
| Giant Foxtail | 0 | 3G | 2G |
| Barnyardgrass | 0 | 2C,5G | 0 |
| Cheatgrass | 0 | 0 | 0 |
| Wild Oats | 0 | 0 | 0 |
| Wheat | 0 | 0 | 0 |
| Corn | 0 | 2C,2G | 0 |
| Barley | 0 | 0 | 0 |
| Soybean | 0 | 0 | 0 |
| Rice | 0 | 0 | 0 |
| Sorghum | 0 | 0 | 0 |
| Sugar beet | 0 | 3G | 5G |
| Cotton | 0 | 0 | 2C |

Table A (continued)

| | Cmpd. 13 | Cmpd. 14 |
|---|---|---|
| Rate kg/ha | 2 | 2 |
| POSTEMERGENCE | | |
| Morningglory | 1B | 0 |
| Cocklebur | 1B | 2G |
| Velvetleaf | 0 | 5G |
| Nutsedge | 0 | 0 |
| Crabgrass | 1B | 0 |
| Giant Foxtail | 1B | 2B |
| Barnyardgrass | 2B | 9C |
| Cheatgrass | 1B | 1B |
| Wild Oats | 0 | 1B |
| Wheat | 0 | 1B |
| Corn | 1B | 1B |
| Barley | 1B | 3B |
| Soybean | 1B | 3B |
| Rice | 1B | 1B |
| Sorghum | 1B | 1B |
| Sugar beet | 1B | 2G |
| Cotton | 0 | 5B |
| PREEMERGENCE | | |
| Morningglory | 0 | 0 |
| Cocklebur | 0 | 0 |
| Velvetleaf | 0 | 0 |
| Nutsedge | 0 | 0 |
| Crabgrass | 4G | 5G |
| Giant Foxtail | 9C | 2C,8G |
| Barnyardgrass | 9C | 10C |
| Cheatgrass | 0 | 0 |
| Wild Oats | 0 | 0 |
| Wheat | 0 | 0 |
| Corn | 0 | 7G |
| Barley | 0 | 0 |
| Soybean | 0 | 0 |
| Rice | 0 | 3G |
| Sorghum | 0 | 6G |
| Sugar beet | 0 | 0 |
| Cotton | 0 | 0 |

Test B

Sixteen cm diameter Wagner pots, equipped with a stoppered drain opening near the bottom of the side wall, were partially filled with Woodstown sandy loam. About 1500 mls of water were added to each pot to bring the water level to a point 3 cm above the soil surface. Japonica and Indica rice seedlings were transplanted as described in Test E. Also, a number of barnyardgrass (Echinochloa crusgalli) seeds were added to each pot. At the same time, seedlings or tubers of the following species were transplanted into the muddy soil: water plantain (Alisma trivale), Scirpus (Scirpus mucranatus), and Cyperus (Cyperus difformis). The weed species selected for this test are of economic importance in major rice-growing areas. The chemical treatments were applied directly to the paddy water after being formulated in a non-phytotoxic solvent within hours after transplanting of two additional species: water chestnut (Eleocharis spp.) and arrowhead (Sagittaria latifolia). Shortly after treatment, the drain hole was opened to drop the water level by two cm. Water was then added to restore the water level to its original height. The following day the draining and refilling process was repeated. The pots were then maintained in the greenhouse. Rates of application and plant response ratings made 21 days after treatment are summarized in Table B. The ratings are percentages where 0 = no injury and 100 = complete kill.

In the subsequent tables, LS is used as an abbreciation for leaf stage.

Table B

Initial Herbicidal Efficacy Tests with Compound 3. Compound was applied to the paddy at the 2.5 LS of Japonica and Indica rice and barnyardgrass at the 0.5 LS.

| Rate (g/ha) | % Injury or Control (average of 2 reports) J | I | BYG | WC | A | SC | CY | WP |
|---|---|---|---|---|---|---|---|---|
| 30 | 0 | 0 | 95 | 0 | 0 | 0 | 0 | 0 |
| 125 | 0 | 0 | 100 | 0 | 0 | 0 | 0 | 0 |

J = Japonica Rice
I = Indica Rice
BYG = Barnyardgrass
WC = Water chestnut
A = Arrowhead
SC = *Scirpus*
CY = *Cyperus*
WP = Water Plantain

Table C

Safety of Compound 3 to Transplanted Japonica and Indica Rice. Compound was applied at the 2.5 LS of rice. The compound was applied as described in Test B.

| Rate (g/ha) | % Injury (average of 2 reports) 0.25 | 1 | 4 | 16 | 63 | 250 | 1000 | 1500 | 2000 |
|---|---|---|---|---|---|---|---|---|---|
| Japonica | 0 | 0 | 0 | 0 | 0 | 25 | 50 | 35 | 50 |
| Indica | 0 | 0 | 0 | 0 | 0 | 0 | 45 | 30 | 30 |

Table D

Efficacy of Compound 3 on Barnyardgrass in Relation to Application Timing and Rate. The compound was applied as described in Test B except at different stages of barnyardgrass growth as shown.

| Application Stage (Leaf Stage) | % Injury (average of 2 reports) g/ha | | | |
|---|---|---|---|---|
| | 30 | 63 | 125 | 250 |
| 0-0.5 | 0 | 83 | 97 | 100 |
| 1.0 | 0 | 60 | 97 | 100 |
| 2.0 | 0 | 0 | 77 | 100 |
| 3.0 | 0 | 0 | 0 | 0 |

Test E

Seeds of rice (Oryza sativa), barnyardgrass (Echinochloa crusgalli), morningglory (Impomoea purpurea), sprangletop (Leptachloa spp.), nutsedge (Cyperus esculentus) and crabgrass (Digitaria ischaemum) were sown into 25 cm diameter plastic pots containing Sassafras sandy loam soil. Compounds, formulated in a non-phytotoxic solvent, were applied as preemergence and postemergence (rice = 2 to 3 leaves) treatments. Evaluation, by visual assessment, were conducted 21 to 25 days following treatment. Weed control and rice injury ratings were based on a % system relative to the untreated controls where 0 = no injury and 100 = complete kill. The results are summarized in Table E.

Table E

Efficacy and Safety of Compound 3 on Upland Rice and Corresponding Weeds

| | Rate | % Injury or Control (average of 2 reports) | | | | | |
|---|---|---|---|---|---|---|---|
| Treatment | Rate (g/ha) | Rice | BYG | NUT | MG | LEP | CRAB |
| Post | 64 | 0 | 0 | 0 | 65 | 0 | 0 |
| | 125 | 0 | 40 | 0 | 90 | 30 | 0 |
| | 250 | 0 | 90 | 0 | 100 | 90 | 0 |
| | 500 | 20 | 85 | 0 | 100 | 100 | 20 |
| Pre | 64 | 0 | 0 | 0 | 0 | 0 | 0 |
| | 125 | 0 | 50 | 35 | 0 | 25 | 0 |
| | 250 | 0 | 100 | 25 | 0 | 95 | 35 |
| | 500 | 35 | 100 | 25 | 0 | 100 | 75 |

Post Application Plant Stages:

| | |
|---|---|
| Rice (Indica): | 1.5 - 2.0 LS |
| BYG (Barnyardgrass): | 2.1 - 2.3 LS |
| Nutsedge (Cyperus esculentus): | 14 - 16 cm |
| MG (Morningglory): | 2.2 - 2.4 LS |
| LEP (Leptochloa): | 2.2 - 2.4 LS |
| Crab (Crabgrass): | 1.5 LS |

Table F

Efficacy and Safety of Compound 3 on Direct-Seeded Indica Rice. Compound applied in a non-phytotoxic solvent either pre- or post-flood.

| | % Injury or Control (average of 2 reports) | | | | | |
|---|---|---|---|---|---|---|
| Rate | Pre-Flood | | | Post-Flood | | |
| (g/ha) | Rice | BYG | SES | Rice | BYG | SES |
| 63 | 0 | 58 | 0 | 0 | 0 | 0 |
| 125 | 23 | 90 | 0 | 0 | 83 | 0 |
| 250 | 63 | 100 | 0 | 0 | 100 | 0 |
| 500 | 83 | 100 | 0 | 12 | 100 | 0 |

Plant Stages:

Pre-Flood: Rice: 0.5 - 1.0 LS
BYG (Barnyardgrass): 1.0 LS
SES (*Sesbania*) Cotyledonary leaves

A permanent flood of 3.0 cm was established 5 days after the pre-flood application.

Post-Flood: Rice: 2.0 LS
BYG: 2.0 LS
SES: 1.0 - 1.5 LS

Table G

Efficacy of 2-[[(4,6-dimethoxypyrimidin-2-yl)aminocarbonyl]aminosulfonylmethyl]benzoic acid (Compound A) + Compound 3 combinations in Transplanted Paddy Rice. The two compounds were applied as described in Test B.

| Treatment | Rate (g/ha) | % Injury or Control (average of 2 reports) J | I | BYG | WC | SC | CY | WP | LPP |
|---|---|---|---|---|---|---|---|---|---|
| Compound | 16 + 63 | 10 | 10 | 95 | 97 | 80 | 90 | 87 | 100 |
| A + | 16 + 250 | 15 | 10 | 100 | 95 | 83 | 95 | 87 | 93 |
| Compound | 30 + 63 | 20 | 7 | 95 | 95 | 83 | 97 | 83 | 100 |
| 3 | 30 + 250 | 20 | 15 | 100 | 100 | 83 | 100 | 97 | 100 |

Abbreviations:
J = Japonica Rice
I = Indica Rice
BYG = Barnyardgrass
WC = Water Chestnut
SC = <u>Scirpus</u>
CY = <u>Cyperus</u>
WP = Water Plantain
LPP = Long-Leaf Pond Plant

Test H - (Herbicidal Activity)

Sixteen cm diameter Wagner pots, equipped with a stoppered drain opening near the bottom of the side wall, were filled with Woodstown sandy loam. About 1500 ml of water were added to each pot to bring the water level to a point 3 cm above the soil surface. Japonica and Indica rice seedlings were transplanted. Also, a number of barnyardgrass (Echinochloa crusgalli) seeds were added to each pot. At the same time, seedlings or tubers of the following species were transplanted into the muddy soil: water plantain (Alisma trivale), Scirpus (Scirpus mucronatus), and Cyperus (Cyperus esculentus). The weed species selected for this test are of economic importance in major rice-growing areas. The chemical treatments were applied within hours after transplanting of two additional species: water chestnut (Eleocharis spp.) and arrowhead (Sagittaria latifolia) plus long-leaf pond plant (Potamogeton spp.) in case of Table 2. Shortly after treatment, the drain hole was opened to drop the water level by two cm. Water was then added to restore the water level to its original height. The following day the draining and refilling process was repeated. The pots were then maintained in the greenhouse. Rates of application and plant response ratings were made 21 days after treatment. The results are in Tables H-1 and H-2.

Table H-1

Initial Herbicidal Efficacy Tests with Compound 7. Compound was applied to the paddy at the 2.5 LS of Japonica and Indica rice and barnyardgrass at the 0.5 LS.

| Rate (g a.i. /ha) | % Injury or Control | | | | | | | | |
|---|---|---|---|---|---|---|---|---|---|
| | J | I | BYG | WC | A | SC | CY | WP | LPP |
| 16 | 0 | 0 | 90 | 0 | 0 | 0 | 0 | 0 | 0 |
| 30 | 0 | 0 | 95 | 0 | 0 | 0 | 0 | 0 | 0 |
| 63 | 0 | 0 | 100 | 0 | 0 | 0 | 0 | 0 | 0 |
| 125 | 0 | 0 | 100 | 0 | 0 | 0 | 0 | 0 | 0 |
| 250 | 0 | 0 | 100 | 0 | 0 | 0 | 0 | 0 | 0 |

J = Japonica Rice
I = Indica Rice
BYG = Barnyardgrass
WC = Water chestnut
A = Arrowhead
SC = _Scirpus_
CY = _Cyperus_
WP = Water Plantain
LPP = Long-Leaf Pond Plant

Table H-2

Efficacy of Londax® + Compound 7 Combinations in Transplanted Paddy Rice.

| Compound | Rate (g a.i./ha) | J | I | BYG | WC | A | SC | CY | WP |
|---|---|---|---|---|---|---|---|---|---|
| Londax® | 16 + 63 | 8 | 0 | 90 | 90 | 40 | 78 | 90 | 85 |
| + | 16 + 125 | 5 | 0 | 95 | 95 | 43 | 85 | 80 | 80 |
| Cmpd. 7 | 16 + 250 | 18 | 5 | 95 | 93 | 50 | 80 | 85 | 75 |
| | 30 + 63 | 10 | 8 | 88 | 93 | 68 | 83 | 95 | 78 |
| | 30 + 125 | 13 | 5 | 93 | 88 | 73 | 83 | 98 | 83 |
| | 30 + 250 | 15 | 10 | 98 | 94 | 80 | 83 | 88 | 83 |
| | 63 + 63 | 25 | 0 | 95 | 93 | 90 | 93 | 98 | 85 |
| | 63 + 125 | 20 | 5 | 90 | 90 | 90 | 88 | 90 | 88 |
| | 63 + 250 | 20 | 13 | 95 | 98 | 90 | 88 | 100 | 88 |

Plant Stages:

J: (Japonica Rice) 2.5 LS
I: (Indica Rice) 2.5 LS
BYG: (Barnyardgrass) 0.5-1.0 LS
WC, A, SC, CY and WP: (Water Chestnut, Arrowhead, (*Scirpus*, *Cyperus* and Water Plantain, respectively). Early postemergence; up to 2-3 cm.

Test I - (Grass Control)

Barnyardgrass and sprangletop (Leptochloa spp.) seeds were sown into 12 cm diameter wax cups containing Sassafras sandy loam soil, which was kept moist but not flooded until seedlings began to emerge. A permanent flood of 3.0 cm depth was established a few hours before each compound treatment.

Following the flood, compounds were applied directly to the paddy after being formulated in a nonphytotoxic solvent.

The weed control was evaluated approximately 2 weeks following compound application. Barnyardgrass and sprangletop control were visually determined using a scale of 0 to 100% (0 = no control; 100 = complete control). Each treatment was replicated twice. The results are in Tables I-1, I-2 and I-3.

Table I-1

Efficacy of Compound 7 on Barnyardgrass.
Compound was applied at the 0.5 LS.

Compound 7

| Rate (g a.i./ha) | | | | | | | | |
|---|---|---|---|---|---|---|---|---|
| 0.25 | 1 | 4 | 16 | 63 | 250 | 1000 | 1500 | 2000 |
| 0 | 0 | 0 | 0 | 68 | 100 | 100 | 100 | 100 |

Table I-2

Efficacy of Compound 7 on Barnyardgrass in Relation to Application Timing and Rate.

| Compound | LS | Rate (g a.i./ha) 30 | 63 | 125 | 250 | 500 | 750 | 1000 |
|---|---|---|---|---|---|---|---|---|
| Cmpd. 7 | 0.5 | 0 | 98 | 100 | 100 | | | |
| | 1 | 0 | 0 | 60 | 98 | | | |
| | 2 | 0 | 0 | 20 | 93 | | | |
| Cmpd. 7 | 2 | | | | 93 | 100 | 100 | 100 |
| | 3 | | | | 0 | 28 | 35 | 30 |
| | 4 | | | | 0 | 10 | 38 | 45 |
| Cmpd. 3 | 2 | | | | 100 | 100 | 100 | 100 |
| | 3 | | | | 0 | 18 | 68 | 63 |
| | 4 | | | | 0 | 10 | 50 | 45 |

LS = Leaf Stage

Table I-3

Efficacy of Compound 7 on Sprangletop in Relation to Application Timing and Rate.

| Compound | LS | Rate (g a.i./ha) 30 | 63 | 125 | 250 | 500 | 750 | 1000 |
|---|---|---|---|---|---|---|---|---|
| Test I | | | | | | | | |
| Cmpd. 7 | 0.5 | 100 | 100 | 100 | 100 | | | |
| | 2 | 0 | 0 | 20 | 100 | | | |
| | 3 | 0 | 0 | 0 | 20 | | | |
| Cmpd. 3 | 0.5 | 38 | 98 | 100 | 100 | | | |
| | 2 | 0 | 0 | 78 | 98 | | | |
| | 3 | 0 | 0 | 0 | 10 | | | |
| Test II | | | | | | | | |
| Cmpd. 7 | 2 | | | | 100 | 100 | 100 | 100 |
| | 3 | | | | 50 | 65 | 98 | 98 |
| | 4 | | | | 18 | 45 | 80 | 78 |
| Cmpd. 3 | 2 | | | | 85 | 93 | 100 | 100 |
| | 3 | | | | 55 | 78 | 85 | 95 |
| | 4 | | | | 20 | 13 | 65 | 60 |

LS = Leaf Stage

Test J - (Upland rice)

Seeds of rice (Oryza sativa), barnyardgrass (Echinochloa crusgalli), morningglory (Impomoea purpurea), sprangletop (Leptochloa spp.), nutsedge (Cyperus esculentus) and crabgrass (Digitaria ischaemum) are sown into 25 cm diameter plastic pots containing Sassafras sandy loam soil. Compounds, formulated in a non-phytotoxic solvent, were applied as preemergence and postemergence (rice = 2.0 leaves) treatments. Evaluation, by visual assessment, is conducted approximately 3 weeks following treatment. Weed control and rice injury ratings were based on a % system relative to the untreated controls.

Table J

Efficacy and Safety of Compound 7 on Upland Rice and Corresponding Weeds.

| | | % Injury or Control | | | | | |
|---|---|---|---|---|---|---|---|
| Treatment | Rate (g a.i./ ha) | RICE | BYG | NUT | MG | LEP | CRAB |
| Pre | 64 | 40 | 0 | 0 | 0 | 0 | 0 |
| | 125 | 0 | 0 | 0 | 95 | 20 | 0 |
| | 250 | 0 | 0 | 0 | 0 | 80 | 0 |
| | 500 | 0 | 100 | 0 | 0 | 95 | 10 |
| Post | 64 | 0 | 0 | 0 | 0 | 0 | 0 |
| | 125 | 0 | 25 | 0 | 0 | 10 | 0 |
| | 250 | 0 | 80 | 0 | 30 | 80 | 0 |
| | 500 | 0 | 100 | 0 | 30 | 95 | 0 |

Plant Stages (Postemergence):

RICE: (Indica) 2.0 LS
BYG: (Barnyardgrass) 3.0 LS
NUT: (Cyperus esculentus)
MG: (Morningglory) 1st True Leaf
LEP: (Leptochloa) 2.5 LS
CRAB: (Crabgrass) 2.5 LS

Test K

Seeds and tubers of rice, barnyardgrass, sesbania, morningglory, velvetleaf, nutsedge, Leptochloa and carly dock were planted into sixteen centimeter diameter airlite pots (two or three species in one pot) containing sassafras sandy loam. When the rice plants started tillering stage (approximately 5 leafstage) compounds, formulated in a non-phytotoxic solvent, were applied as overhead spray. The pots were then floated with 3 cm of water and kept that way until the final evaluation.

Evaluation, by visual assessment, was conducted approximately three weeks following treatment.

Table K

Efficacy and Safety of Compound 7 with Late Post Foliar Application.

| Compound | Rate (g a.i./ ha | % Injury or Control | | | | | | | |
|---|---|---|---|---|---|---|---|---|---|
| | | RICE | BYG | SES | MG | VL | NUT | LEP | CD |
| Cmpd. 7 | 32 | 0 | 0 | 0 | 0 | 0 | 0 | 0 | 10 |
| | 64 | 0 | 0 | 0 | 0 | 0 | 85 | 0 | 20 |
| | 125 | 0 | 0 | 0 | 0 | 0 | 0 | 0 | 0 |
| | 250 | 0 | 0 | 0 | 0 | 0 | 0 | 0 | 0 |
| Cmpd. 3 | 32 | 0 | 0 | 0 | 0 | 0 | 0 | 0 | 0 |
| | 64 | 0 | 0 | 0 | 0 | 0 | 0 | 65 | 40 |
| | 125 | 0 | 20 | 0 | 0 | 0 | 25 | 90 | 25 |
| | 250 | 0 | 90 | 0 | 0 | 0 | 70 | 100 | 0 |

Plant Stages:

RICE: (Indica) 4.5-5 LS, Tillering
BYG: (Barnyardgrass) 30 cm
SES: (*Sesbania*) 30-50 cm
MG: (Morningglory) 50-55 cm
VL: (Velvetleaf) 8-30 cm, inconsistent growth
NUT: (*Cyperus esculentus*) 11-15 cm
LEP: (*Leptochloa*) 6-12 cm
CD: (Carly dock) 8-10 cm

Claims: BA-8585-A

1. Compounds of the Formula I

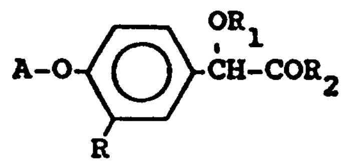

I

where

A is 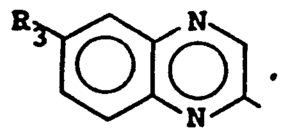 , 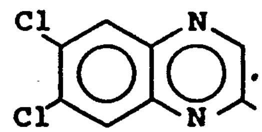 , 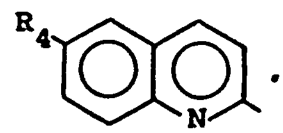 ,

A-1 A-2 A-3

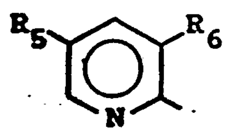 , 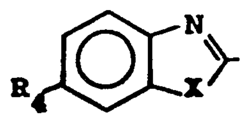 or 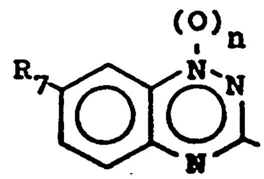 ;

A-4 A-5 A-6

X is O or S;

n is 0 or 1;

R is H, Cl, F, Br, $CH_3$, $OCH_3$ or $OCH_2CH_3$;

$R_1$ is $C_1$-$C_3$ alkyl, allyl or propargyl;

$R_2$ is $X_1R_8$, OH;

$R_3$ is Cl, F, Br or $CF_3$;

$R_4$ is H, Cl, F or Br;

$R_5$ is Cl or $CF_3$;

$R_6$ is H or Cl;

$R_7$ is H, Cl, F, Br or $CF_3$;

$R_8$ is $C_1$-$C_4$ alkyl, benzyl, phenyl, $C_5$-$C_8$ cycloalkyl, $CH_2CH_2OCH_3$, $CH_2CH_2OCH_2CH_3$, $C_3$-$C_4$ alkenyl, $C_3$-$C_4$ alkynyl, $C_3$-$C_4$ alkenyl substituted with Cl, $CH_2CH_2OCH_2CH_2OCH_3$, $CH_2CH_2OCH_2CH_2OCH_2CH_3$, $CH_2CO_2CH_3$ or $CH(CH_3)CO_2CH_3$;

$X_1$ is O or S;

provided that

a) when $R_5$ is Cl, then $R_6$ is Cl;

b) when A is $A_2$, then R is H;

and their agriculturally suitable salts.

2. Compounds of Claim 1 wherein $R_1$ is $CH_3$ and $R_2$ is $XR_8$.

3. Compounds of Claim 2 wherein A is A-1, A-3, A-4 or A-5; R is H, Cl, $OCH_3$ or F; and $R_8$ is $C_1$-$C_4$ alkyl.

4. Compounds of Claim 3 wherein A is A-1.

5. Compounds of Claim 2 wherein A is A-2, R is H, Cl, $OCH_3$ or F; and $R_8$ is $C_1$-$C_4$ alkyl.

6. Compounds of Claim 2 wherein A is A-6, R is H, Cl, $OCH_3$ or F; and $R_8$ is $C_1$-$C_4$ alkyl.

7. Compounds of Claim 3 wherein A is A-5.

8. Compounds of Claim 3 wherein A is A-3.

9. Compounds of claim 1 or claim 2 wherein R is H, F, Cl, Br, $OCH_3$ or $OCH_2CH_3$.

10. The compound of Claim 1 which is 4-(6-chloro-2-quinoxalinyloxy)-3-fluoro-α-methoxybenzene-acetic acid, methyl ester.

11. The compound of Claim 1 which is 4-(6-chloro-2-quinoxalinyloxy)-α-methoxybenzeneacetic acid, methyl ester.

12. The compound of Claim 1 which is 3-chloro-4-(6-chloro-2-quinoxalinyloxy)-α-methoxybenzeneacetic acid, methyl ester.

13. The compound of Claim 1 which is 4-(6-chloro-2-quinoxalinyloxy)-α-3-dimethoxybenzeneacetic acid, methyl ester.

14. An agriculturally suitable composition for controlling the growth of undesired vegetation comprising an effective amount of a compound of any of claims 1 to 13 and at least one of the following: surfactant, solid or liquid diluent.

15. **A method for controlling the growth of undesired vegetation which comprises applying to the locus to be protected an effective amount of a compound of** any of claims 1 to 13.

16. A method of claim 15 wherein said undesired vegetation is selectively controlled in rice, soy beans cotton or sugar beet.

17. A process for the preparation of a compound of claim 1 which comprises:

(a) reacting a haloheterocycle of formula

A - Hal

with an alkali metal salt of a 4-hydroxybenzeneacetate ester of formula

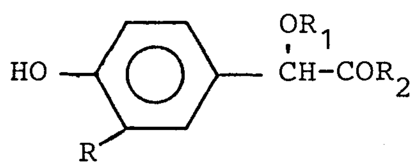

wherein $R_1$ is $C_1$-$C_3$ alkyl and $R_2$ is $OC_1$-$C_3$ alkyl;

(b) alkylating a compound of formula

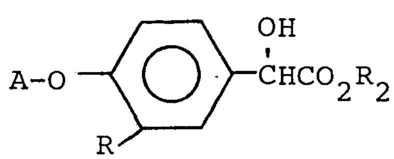

wherein $R_2$ is $OC_1$-$C_3$ alkyl, to introduce a desired group $R_1$ by methods known per se;

(c) hydrolysing a compound of formula (I) wherein $R_2$ is $OC_1$-$C_3$ alkyl to obtain a compound of formula (I) wherein $R_2$ is OH; or

(d) esterifying an acid of formula (I) wherein $R_2$ is OH by methods known per se to obtain an ester of formula (I) wherein $R_2$ is $X_1R_8$.

For the designated contract state AT

60

Claims: BA-8585-A

1. A process for the preparation of compounds of the Formula I

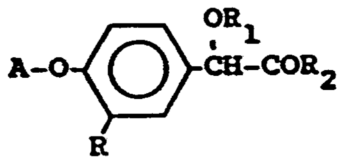

I

where

A is 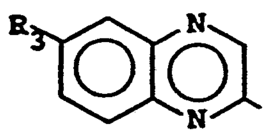 , 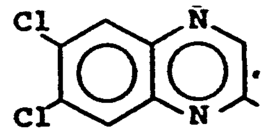 , 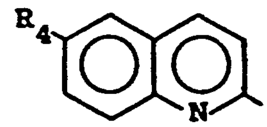 ,

A-1 A-2 A-3

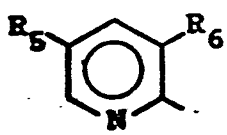 , 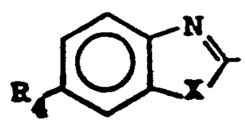 or 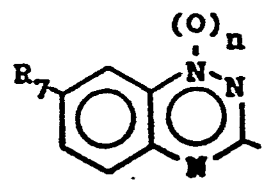 ;

A-4 A-5 A-6

X is O or S;

n is O or 1;

R is H, Cl, F, Br, $CH_3$, $OCH_3$ or $OCH_2CH_3$;

$R_1$ is $C_1$-$C_3$ alkyl, allyl or propargyl;

$R_2$ is $X_1R_8$, OH;

$R_3$ is Cl, F, Br or $CF_3$;

$R_4$ is H, Cl, F or Br;

$R_5$ is Cl or $CF_3$;

$R_6$ is H or Cl;

$R_7$ is H, Cl, F, Br or $CF_3$;

$R_8$ is $C_1$-$C_4$ alkyl, benzyl, phenyl, $C_5$-$C_8$ cycloalkyl, $CH_2CH_2OCH_3$, $CH_2CH_2OCH_2CH_3$, $C_3$-$C_4$ alkenyl, $C_3$-$C_4$ alkynyl, $C_3$-$C_4$ alkenyl substituted with Cl, $CH_2CH_2OCH_2CH_2OCH_3$, $CH_2CH_2OCH_2CH_2OCH_2CH_3$, $CH_2CO_2CH_3$ or $CH(CH_3)CO_2CH_3$;

$X_1$ is O or S;

provided that

a) when $R_5$ is Cl, then $R_6$ is Cl;

b) when A is $A_2$, then R is H;

and their agriculturally suitable salts;

which comprises:

(a) reacting a haloheterocycle of formula

A - Hal

with an alkali metal salt of a 4-hydroxybenzeneacetate ester of formula

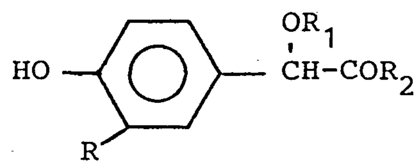

wherein $R_1$ is $C_1$-$C_3$ alkyl and $R_2$ is $OC_1$-$C_3$ alkyl;

(b) alkylating a compound of formula

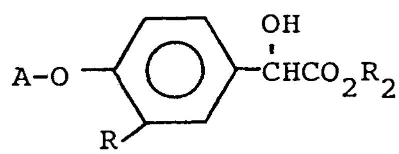

wherein $R_2$ is $OC_1$-$C_3$ alkyl, to introduce a desired group $R_1$ by methods known per se;

(c) hydrolysing a compound of formula (I) wherein $R_2$ is $OC_1$-$C_3$ alkyl to obtain a compound of formula (I) wherein $R_2$ is OH; or

(d) esterifying an acid of formula (I) wherein $R_2$ is OH by methods known per se to obtain an ester of formula (I) wherein $R_2$ is $X_1R_8$.

2. A process of Claim 1 wherein $R_1$ is $CH_3$ and $R_2$ is $XR_8$.

3. A process of Claim 2 wherein A is A-1, A-3, A-4 or A-5; R is H, Cl, $OCH_3$ or F; and $R_8$ is $C_1$-$C_4$ alkyl.

4. A process of Claim 3 wherein A is A-1.

5. A process of Claim 2 wherein A is A-2, R is H, Cl, $OCH_3$ or F; and $R_8$ is $C_1$-$C_4$ alkyl.

6. A process of Claim 2 wherein A is A-6, R is H, Cl, $OCH_3$ or F; and $R_8$ is $C_1$-$C_4$ alkyl.

7. A process of Claim 3 wherein A is A-5.

8. A process of Claim 3 wherein A is A-3.

9. A process of claim 1 or claim 2 wherein R is H, F, Cl, Br, $OCH_3$ or $OCH_2CH_3$.

10. The process of Claim 1 wherein the product is 4-(6-chloro-2-quinoxalinyloxy)-3-fluoro-α-methoxybenzeneacetic acid, methyl ester, or an agriculturally suitable salt thereof.

11. The process of Claim 1 wherein the product is 4-(6-chloro-2-quinoxalinyloxy)-α-methoxybenzeneacetic acid, methyl ester, or an agriculturally suitable salt thereof.

12. The process of Claim 1 wherein the product is 3-chloro-4-(6-chloro-2-quinoxalinyloxy)-α-methoxybenzeneacetic acid, methyl ester, or an agriculturally suitable salt thereof.

13. The process of Claim 1 wherein the product is 4-(6-chloro-2-quinoxalinyloxy)-α-3-dimethoxybenzeneacetic acid, methyl ester, or an agriculturally suitable salt thereof.

14. An agriculturally suitable composition for controlling the growth of undesired vegetation comprising an effective amount of a compound of formula (I) as defined in any of claims 1 to 13 and at least one of the following: surfactant, solid or liquid diluent.

15. A method for controlling the growth of undesired vegetation which comprises applying to the locus to be protected an effective amount of a compound of formula (I) as defined in any of claims 1 to 13.

16. A method of claim 15 wherein said undesired vegetation is selectively controlled in rice, soy beans cotton or sugar beet.